(19) Europäisches Patentamt
European Patent Office
Office européen des brevets

(11) EP 1 408 032 B1

(12) # FASCICULE DE BREVET EUROPEEN

(45) Date de publication et mention de la délivrance du brevet:
**16.02.2011 Bulletin 2011/07**

(51) Int Cl.:
***A61Q 17/04*** *(2006.01)* ***C07D 209/16*** *(2006.01)*
***A61K 31/404*** *(2006.01)* ***C07D 403/12*** *(2006.01)*
***C07D 209/20*** *(2006.01)*

(21) Numéro de dépôt: **03022092.5**

(22) Date de dépôt: **02.10.2003**

(54) **Produit de couplage entre la tryptamine et un alpha-aminoacide et son application dans le domaine de la neuro-cosmétique**

Kupplungsprodukt zwischen Tryptamin und eine alpha-Aminosaüre und deren Verwendung im neurokosmetikischen Bereich

Coupling product between tryptamine and an alpha-amino acid and its use in the field of neuro-cosmetics

(84) Etats contractants désignés:
**AT BE BG CH CY CZ DE DK EE ES FI FR GB GR HU IE IT LI LU MC NL PT RO SE SI SK TR**

(30) Priorité: **04.10.2002 MC 2488**

(43) Date de publication de la demande:
**14.04.2004 Bulletin 2004/16**

(73) Titulaire: **EXSYMOL S.A.M.**
**MC-98000 Monte Carlo (MC)**

(72) Inventeur: **Seguin, Marie-Christine**
**98000 Monaco (MC)**

(74) Mandataire: **de Zeeuw, Johan Diederick**
**Murgitroyd & Company,**
**Immeuble Atlantis**
**55, Allée Pierre Ziller,**
**Sophia Antipolis**
**06560 Valbonne (FR)**

(56) Documents cités:
**EP-A- 1 020 179**
**WO-A-00/72815**
**WO-A-94/19325**
**WO-A-95/12581**
**WO-A-03/072124**

- **PATRICIA MELNYK ET AL.: "A new diastereoselective synthesis of cis 1-aminoindoloquinolizidine" TETRAHEDRON LETTERS., vol. 34, no. 32, - 1993 pages 5085-5088, XP002271986 ELSEVIER SCIENCE PUBLISHERS, AMSTERDAM., NL ISSN: 0040-4039**
- **MUELLER, J. CONSTANZE D. ET AL.: "Non-peptidic cysteine derivatives as inhibitors of matrix metalloproteinases" BIOLOGICAL CHEMISTRY, vol. 378, no. 12, - 1997 pages 1475-1480, XP008028085**
- **JEAN-FRANCOIS NICOLAy, ISABELLE IMBERT: "Targeting the cutaneous nervous network" COSMETICS & TOILETRIES, vol. 118, no. 7, - 2003 pages 37-40,42,44, XP008028069**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 408 032 B1

Printed by Jouve, 75001 PARIS (FR)

## Description

La présente invention a pour objet une famille de pseudodipeptides, produits de couplage entre la tryptamine, amine primaire de nature indolique, et une sélection d'alpha-aminoacides.

L'objet de l'invention concerne également le procédé de préparation desdits produits, ainsi que leurs applications en tant que substances actives sur le système nerveux cutané.

Les intéractions entre système nerveux et cellules cutanées, tant sur les plans anatomique que fonctionnel, sont nombreuses et désormais bien établies. Cette récente compréhension a d'ailleurs ouvert la porte à divers champs d'activité, en particulier en cosmétique, qualifiée de "neuro-cosmétique" pour traduire toute action qui vise à agir sur de telles intéractions, et en conséquence, à remédier à toute altération ou "désordre" cosmétique cutané inhérent.

La peau est en effet un organe particulièrement innervé, avec une innervation dense et fine dans les couches dermiques, mais également jusqu'aux couches les plus superficielles situées dans l'épiderme, à l'exception de la couche cornée. Cette innervation est notamment à la base de tout notre système sensoriel, tel les sensations de toucher, douleur, démangeaison, température, pression, etc....

Les connexions entre nerfs et peau sont ainsi très étroites, caractérisées par, outre des contacts physiques, un échange d'informations permanent entre les cellules nerveuses et les cellules cutanées. Les mécanismes, à l'origine de cette communication dite "neurogénique", sont désormais connus.

Ces échanges sont le fruit, tout d'abord, de substances biologiquement actives que sont les neuromédiateurs (Lotti T. et al., J. Am. Acad. Dermatol. (1995), vol.33, pp.482-496). La plupart de ces vecteurs chimiques de l'information nerveuse retrouvés dans le derme et l'épiderme sont de nature peptidique : substance P, neuropeptide Y, calcitonin gene-related peptide ou CGRP, etc.... Mais d'autres appartiennent au groupe des catécholamines avec notamment l'adrénaline et l'acétylcholine.

D'autre part, ces échanges résultent également de l'existence, à la surface des cellules de la peau, nerveuses ou pas, de récepteurs spécifiques des neuromédiateurs qui, lorsqu'activés par ces derniers, modulent les propriétés des cellules cutanées, aussi bien épidermiques (kératinocytes, mélanocytes, cellules de Langerhans) que dermiques (fibroblastes, cellules endothéliales).

D'une façon générale, il est maintenant clairement admis une forte implication du système nerveux dans les métabolismes cutanés.

Toutes les principales fonctions de la peau, telles l'immunité, la défense de l'organisme contre les agressions du milieu extérieur, la différentiation et la prolifération cellulaire, la pigmentation, sont aujourd'hui susceptibles d'être modulées, voire contrôlées, par le système nerveux (L. Misery, International Journal of Cosmetic Science (2002), vol. 24, pp.111-116).

Au niveau de la peau et de son rôle dans le mécanisme immunitaire par exemple, une altération du système nerveux cutané, après l'agression d'un corps étranger localisé, s'accompagne d'une réaction inflammatoire anormale. En effet, les neuropeptides cutanés, sécrétés par les terminaisons nerveuses, participent aux mécanismes de cette réaction inflammatoire en agissant sur les récepteurs membranaires des cellules immunes (lymphocytes, macrophages) et/ou cutanées (kératinocytes, mélanocytes, fibroblastes, cellules de Langerhans) afin de libérer les cytokines, substances nécessaires, selon les types, à l'induction, l'entretien ou la réduction de l'état inflammatoire. Le neuropeptide "substance P" est ainsi décrit être un activateur de la synthèse de cytokines (IL-1 ou TNF-alpha (Ansel J.C. et al., Journal of Investigative Dermatology Symposium Proceedings (1997), vol.2, pp.23-26).

Un autre neuropeptide, le CGRP ou "calcitonin gene-related peptide", est lui plutôt considéré comme un stimulant de la prolifération kératinocytaire (Takahashi K. et al., J. Invest. Dermatol. (1993), vol.101, pp.646-651).

En conséquence, il est aujourd'hui perçu tout l'intérêt d'intervenir sur les cellules nerveuses dans la biologie cutanée. Les applications potentielles de cette implication sont ainsi nombreuses en cosmétologie. De nouvelles perspectives s'offrent notamment dans le traitement de certaines altérations de la peau, telles la neurodégénérescence cutanée, les phénomènes d'inflammation et d'irritation, les troubles de la desquamation, le vieillissement et la sécheresse cutanée, la cicatrisation, les dermatoses du visage, la sudation excessive, etc... (L. Misery, International Journal of Cosmetic Science (2002), vol.24, pp.111-116 et références citées).

La demanderesse a donc considéré une approche qui vise à agir sur certaines fonctions biologiques de la peau impliquant le système nerveux, mais de manière locale exclusivement, c'est-à-dire sur les seules terminaisons nerveuses de la peau, et non pas, à l'instar de nombreuses applications thérapeutiques, sur le système nerveux central. Il n'est nullement envisagé en outre une action au niveau cérébral accompagnée d'une répercussion cutanée.

A cet effet, la demanderesse a porté son choix sur l'utilisation d'un type d'actif, acceptable en cosmétique, de structure voisine des substances neurogènes naturelles identifiées pour régir les intéractions entre terminaisons nerveuses et cellules cutanées, et capable d'interférer sur ces communications nerveuses cutanées. Elle a aussi considéré un désordre cosmétique induit par une situation de stress ou de privation de facteur de croissance, exposées et détaillées ci-après dans la description de l'invention.

Ainsi, par analogie avec les neuromédiateurs retrouvés dans la peau, et plus particulièrement les neuropeptides,

la demanderesse a retenu une structure de nature peptidique ou proche de celle-ci. Elle a pour cela opté pour un panel d'alpha-aminoacides naturels propres à constituer un neuropeptide. Parmi ce panel, elle a sélectionné un type d'aminoacides à chaîne latérale polaire ou apolaire, au comportement chélateur de métaux et à l'activité antioxydante (Ahmad M. M. et al., JAOCS (1993), vol.80, pp.837-840), (Gopala Krishna A.G. et al., JAOCS (1994), vol.71, pp.645-647), Popov I. et al., Luminescence (1999), vol.14, pp.169-174), en raison de la nature oxydative des nombreux stress responsables des altérations cutanées et des résultats obtenus par la demanderesse avec certains aminoacides de cette sélection dans la mise en évidence des propriétés neuro-cométiques.

Enfin, afin de cibler l'actif vers la cellule nerveuse, la demanderesse a également sélectionné la présence d'un motif indolique puisqu'il existe, à la surface des cellules nerveuses, des récepteurs membranaires dont l'affinité pour ce type de motif moléculaire est aujourd'hui connue.

L'objet de l'invention est par conséquent une famille de pseudodipeptides résultant du couplage entre la tryptamine, amine primaire possédant un noyau indole, et une sélection d'alpha-aminoacides, lesdits pseudodipeptides étant représentés par la formule générale (I) suivante :

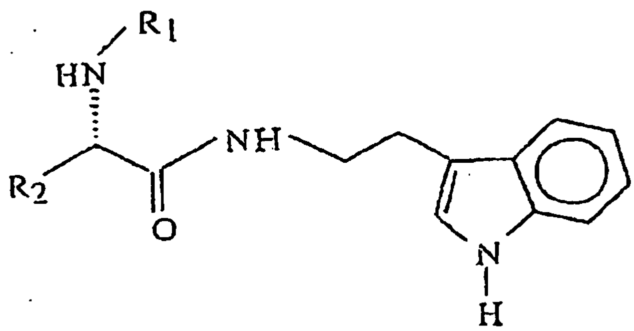

(I)

dans laquelle:

$R_1$ représente un atome d'hydrogène, un radical acyle ou, alkyloxycarbonyl,

$R_2$ représente la chaîne latérale d'un alpha-aminoacide choisi parmi l'acide L-glutamique, la L-arginine, la L-méthionine, la L-histidine, le L-tryptophane, la L-tyrosine.

Il est à noter que lorsque $R_1$ représente un radical acyle ou acyloxy, substituants biodégradables pouvant être hydrolysés *in vivo,* les dérivés correspondants constituent des formes précurseurs des pseudodipeptides ciblés, au caractère lipophile propre à favoriser leur pénétration cutanée, et donc à améliorer leur biodisponibilité après application topique dudit pseudodipeptide.

De manière avantageuse, on citera les pseudodipeptides alpha-L-glutamyltryptamine, L-methionyltryptamine et L-tryptophantryptamine, l'exemple préféré étant l'alpha-L-glutamyltryptamine.

Dans le cas du pseudodipeptide alpha-L-glutamyltryptamine, l'invention concerne également l'utilisation d'un analogue, doué des mêmes propriétés que ce dernier, résultant de la conversion du radical glutamique en radical pyroglutamique selon une cyclisation intramoléculaire bien connue de l'état de la technique (Burstein Y. et al., Proc. Natl. Acad Sci. USA (1976), vol.73, pp.2604-2608) et de l'homme du métier en tant que médicament destiné à ralentir le processus de neurodégenerecence cutané par traitement local.

Une autre réalisation préférée de l'invention est le pseudodipeptide de formule générale (I) dans lequel $R_1$ représente un radical acétyle ou ter-butyloxycarbonyle, et $R_2$ représente la chaîne latérale d'un alpha-aminoacide choisi parmi l'acide L-glutamique, la L-méthionine, le L-tryptophane.

A ce jour, et à la connaissance de la demanderesse, les structures visées par celle-ci sont nouvelles puisque nullement divulguées. L'état antérieur de la technique révèle certes des structures voisines, notamment avec les demandes EP-A-1 020 179 et WO 94/19325 mais jamais aux fins susmentionnées et selon l'approche considérée.

La littérature divulgue en effet un certain nombre de dérivés aminoacyl d'une amine dite "biogène", car synthétisée dans l'organisme, à caractère indolique: la sérotonine ou 5-hydroxytryptamine. Cette amine primaire provenant de l'hydroxylation et de la décarboxylation de l'acide aminé essentiel tryptophane est à la fois un médiateur chimique dans le système nerveux central et une neurohormone déversée dans les voies sanguines et urinaires (Vigy M., Conc. Méd. (1969), vol.14, pp.2865-2868). Elle intervient dans de nombreux domaines (Hindle A.T., Br. J. Anaesth. (1994), vol.73, pp.395-407) et notamment dans le mécanisme de divers troubles psychiatriques (dépression, schizophrénie, anxiété, etc) ainsi que dans certaines pathologies neurologiques comme la maladie d'Alzheimer ou la migraine.

Dans le but de réduire la neurotoxicité accompagnant son utilisation pharmacologique mais aussi la multiplicité

de ses effets, des résidus aminoacides ont été conjugués à la sérotonine ou à son analogue méthoxylé. Il est ainsi décrit les synthèses des L-Gly-5-hydroxytryptamine, beta-L-Ala-5-hydroxytryptamine, gamma-L-aminobutyryl-5-hydroxytryptamine, L-Met-5-hydroxytryptamine, alpha-L-Glu-5-hydroxytryptamine, L-Cyst-5-hydroxytryptamine (Suvorov N.N. et al., Bioorg. Khim. (1976), vol.2, pp.729-736), des L-Gly-5-methoxytryptamine, alpha-L-Ala-5-methoxytryptamine, beta-L-Ala-5-methoxytryptamine, gamma-L-Glu-5-methoxytryptamine, L-Arg-5-methoxytryptamine, L-Val-5-methoxytryptamine, L-Meth-5-methoxytryptamine, L-Trp-5-methoxytryptamine, L-Cyst-5-methoxytryptamine (Popova G. V. et al., Tr. Mosk. Khim. Tekhnol. Inst. im D I Mendeleeva (1977), vol.94, pp.84-98), la synthèse du alpha-L-Glu-5-methoxytryptamine (Popova G.V. et al., Zh. Obshch. Khim. (1979), vol.49, pp.1418-1424). Dans les composés ci-dessus, et également par la suite, les résidus aminoacides impliqués dans la liaison avec l'amine primaire sont représentés par leur code à trois lettres, selon la nomenclature ci-après

| | |
|---|---|
| Gly | glycine |
| Ala | alanine |
| Met | méthionine |
| Glu | acide glutamique |
| Arg | arginine |
| Val | valine |
| Trp | tryptophane |
| Cyst | cystéine |

Le brevet SU 296409 concerne la préparation de dérivés peptidiques de la sérotonine et de la 5-methoxytryptamine. Le document rapporte des propriétés radioprotectrices pour toutes ces structures.

Avec l'alpha-méthyltryptamine, un autre analogue de la sérotonine est lui aussi connu de longue date. Médicalement étudié en tant qu'antidépresseur potentiel (Mashkovskii M.D. et al., Psikhiatr. (1963), n°1, pp.72), il a même été commercialisé dans les années soixante en Union Soviétique sous le nom d'Indopan® et revendiquait, outre une activité antidépressive, une action stimulante sur le système nerveux central avec notamment une stimulation de l'activité motrice et de l'excitabilité des réflexes. Mais toujours dans l'optique de moduler les propriétés indésirables de l'alpha-méthyltryptamine, il a été introduit par la suite un résidu aminoacide sur la chaîne latérale de l'amine, notamment l'acide glutamique (Vigdorchik M.M. et al., Pharm. Chem. J. (1977), vol.11, pp.305-309), les propriétés pharmacologiques du alpha-L-glutamyl-DL-alpha-methyltryptamine résultant étant alors comparées avec l'Indopan®.

L'homologue glutamique alpha-éthylé fut également synthétisé (Bulatova N.N. et al., Khim. Farm. Zh. (1968), vol.2, pp.6-9), et son action sur le système nerveux central comparée à celle du alpha-L-glutamyl-DL-alpha-methyltryptamine.

La demanderesse ne se place nullement dans ce contexte de l'art antérieur, à savoir une action directe sur le système nerveux central, ni dans celui d'une amélioration, par une tolérance accrue et un effet plus important dans le temps, des propriétés pharmacologiques des indoleamines sérotonine ou alpha- méthyltryptamine. Dans une toute autre approche, la demanderesse a envisagé la synthèse d'une substance active capable, au regard de son analogie structurale avec les neuromédiateurs cutanés, de marquer une affinité pour les récepteurs des cellules nerveuses et cutanées afin d'induire certaines propriétés neuro-cosmétiques, exposées ci-après avec la présentation des tests.

Dans l'état de la technique, il est également à noter l'identification de glutamylamines, dont la glutamyltryptamine, chez le mollusque marin *Aplysia californía.* Dans tous les cas, il n'a été isolé puis chimiquement reproduit que des dérivés glutamiques conjugués en position gamma avec les amines tryptamine, hydroxytryptamine, dopamine, octopamine, tyramine et phenylethylamine (Mc Caman M.W. et al., J. Neurochem. (1985), vol.45, 1828-1835), l'étape de gamma-glutamylation desdites amines étant supposée inactiver ces dernières.

Parmi les produits répondant à la formule générale (I), les exemples ci-après constituent une liste non limitative des pseudodipeptides selon l'invention :

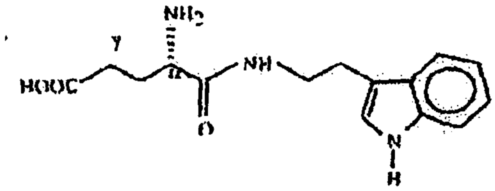

alpha-L-glutamyltryptamine
(alpha-L-Glu-Tryp)

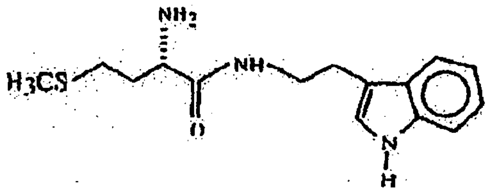

L-methionyltryptamine
(L-Met-Tryp)

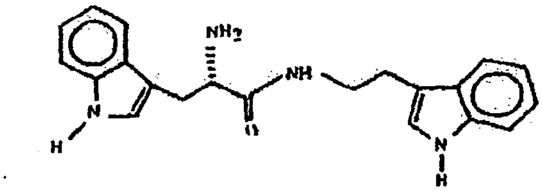

L-tryptophantryptamine
(L-Trp-Tryp)

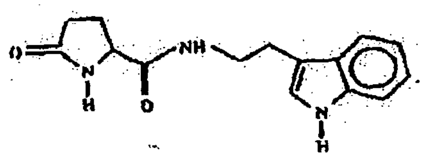

L-pyroglutamyltryptamine
(L-pGlu-Tryp)

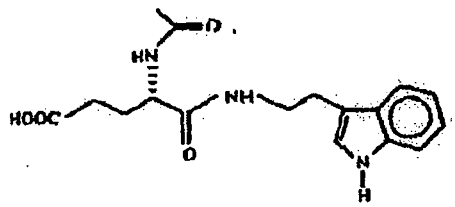

N-acetyl-alpha-L-glutamyltryptamine
(N-Ac-alpha-L-Glu-Tryp)

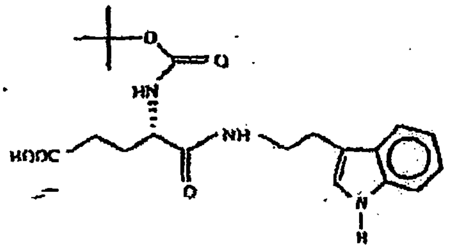

N-ter-butyloxycarbonyl-alpha-L-glutamyltryptamine
(N-Boc-alpha-L-Glu-Tryp)

La présente invention concerne en outre un procédé chimique mis au point pour la préparation des pseudodipeptides objets de l'invention. I1 comporte successivement les étapes suivantes

La première étape consiste à protéger la fonction alpha-amino du L-aminoacide par un radical acyle ou acyloxy,

préférentiellement par les radicaux acétyle ou ter-butyloxycarbonyle.

Dans le cas de l'acide glutamique, l'étape de protection de la fonction alpha-amino est suivie immédiatement d'une étape d'estérification de la fonction gamma-carboxylique par un radical alkyle, préférentiellement par le radical ter-butyle.

La seconde étape du procédé consiste à coupler le L-aminoacide N-protégé et, dans le cas de l'acide-L-glutamique, gamma-O-estérifié, à la tryptamine. Ce couplage s'effectue soit directement à l'aide d'un agent de couplage classique, préférentiellement le N, N'-dicyclohexylcarbodiimide, soit via l'activation préalable ou *in situ* de la fonction alpha-carboxylique de l'aminoacide N-protégé par action d'un activateur classique, préférentiellement l'hydroxybenzotriazole, l'expression "classique" signifiant un agent bien connu de l'homme de l'art.

Dans une troisième étape, facultative selon le pseudodipeptide recherché, le groupement N-protecteur du pseudodipeptide résultant de l'étape susmentionnée est éliminé, avantageusement par acidolyse et préférentiellement à l'aide d'une solution aqueuse d'acide chlorhydrique.

L'invention a aussi pour objet des compositions neuro-cosmétiques contenant, à titre de principe actif, un pseudodipeptide de formule générale (I), préférentiellement l'alpha-L-glutamyltryptamine, en association avec un ou plusieurs excipients cosmétiquement appropriés.

Un dernier objet de l'invention vise l'utilisation neuro-cosmétique des pseudodipeptides selon l'invention. Celle-ci découle des propriétés présentées ci-après mettant en évidence la capacité desdits pseudodipeptides à interagir sur des cellules nerveuses cutanées.

La demanderesse a ainsi démontré l'utilisation des pseudodipeptides selon l'invention successivement comme agent neuro-cosmétique présentant un effet cytoprotecteur, autrement désigné neuroprotecteur, vis-à-vis de cellules nerveuses cutanées soumises à un rayonnement ultra-violet, comme agent neuro-cosmétique destiné à ralentir le processus de neurodégénérescence, comme agent neuro-cosmétique destiné à lutter contre l'inflammation neurogène, et comme agent neuro-cosmétique capable de stimuler les cellules immunes cutanées.

Le modèle cellulaire choisi par la demanderesse dans toutes ses expérimentations *in vitro* a été une lignée cellulaire phéochromocytomale d'origine murine, appelée "PC 12", communément acceptée pour des études neurobiologiques et neurochimiques sur cellules nerveuses (Greene L.A. et al., Proc. Natl. Acad. Sci. USA (1976), vol.73, pp. 2424-2428), et en particulier sur les neurones périphériques qui innervent la peau (Keilbaugh S.A., Biochem. Pharm. (1997), vol.53, pp.1485-1492).

La lignée PC 12 est utilisée après différentiation, selon une procédure de la littérature (Greene L.A. et al. in 'Culturing Nerve Cells' (1991), MIT Press, Cambridge, MA, pp.207-225).

Les tests suivants illustrent les propriétés ou effets susmentionnés.

Test 1 : effet cytoprotecteur de l'alpha-L-glutamyltryptamine, du L-methionyltryptamine et du L-tryptophantryptamine vis à vis de PC 12 soumises à un stress UV-B. Comparaison avec un antioxydant de référence.

Un stress UV-B cytotoxique est appliqué sur le modèle de cellules nerveuses (285 nm $\pm$ 5; 500 mJ/cm$^2$), en absence puis en présence d'actif, successivement les alpha-L-glutamyltryptamine (Glu-Tryp), L-methionyltryptamine (Met-Tryp) et L-tryptophantryptamine (Trp-Tryp).

La mort cellulaire est ensuite évaluée par la mesure de l'activité lactico-deshydrogénase (LDH) dans le milieu de culture. Cette activité est proportionnelle à la lyse cellulaire qui suit la mort cellulaire.

Les résultats sont exprimés en % de protection donné par le rapport des activités LDH, selon l'équation suivante:

$$\%\ \text{protection} = \frac{LDH_{\text{cellules traitées}} - LDH_{\text{cellules témoins non traitées}}}{LDH_{\text{cellules témoins non traitées}}} * 100$$

Les résultats sont comparés à ceux obtenus avec un antioxydant de référence, la vitamine E (Vit.E).

La validité du test est vérifiée par la mesure de l'activité LDH dans le milieu de culture de cellules non stressées (contrôle négatif). Les valeurs figurant dans les tableaux ci-après sont des valeurs moyennes obtenues à partir de 6 mesures.

RESULTATS :

| | Glu-Tryp (1,72 mM) | Glu-Tryp (0,86 mM) | Glu-Tryp (0,43 mM) | Glu-Tryp (0,1 mM) | Glu-Tryp (0,05 mM) | VitE (2 mM) |
|---|---|---|---|---|---|---|
| % de protection | 69 | 61 | 48 | 39 | 26 | 34 |

| | Met-Tryp (1,91 mM) | Met-Tryp (0,85 mM) | Met-Tryp (0,48 mM) | Met-Tryp (0,1 mM) | Met-Tryp (0,05 mM) | VitE (2 mM) |
|---|---|---|---|---|---|---|
| de protection | 65 | 53 | 42 | 32 | 20 | 34 |

| | Trp-Tryp (1,80 mM) | Trp-Tryp (0,85 mM) | Trp-Tryp (0,45 mM) | Trp-Tryp (0,1 mM) | Trp-Tryp (0,05 mM) | VitE (2 mM) |
|---|---|---|---|---|---|---|
| % de protection | 66 | 58 | 45 | 35 | 22 | 34 |

Test 2 : Effet anti-âge de l'alpha-L-glutamyltryptamine, du L-methionyltryptamine et du L-tryptophantryptamine avec le ralentissement du processus de neurodégénérescence de PC 12 soumises à une privation de sérum

Une privation de sérum est appliquée aux PC 12 afin de reproduire les effets du vieillissement. Le processus de neurodégénérescence est suivi, en absence puis en présence d'actif, successivement les alpha-L-glutamyltryptamine (Glu-Tryp), L-methionyltryptamine (Met-Tryp) et L-tryptophantryptamine (Trp-Tryp), par une mesure cinétique de la libération dans le milieu de culture de l'enzyme lactico-deshydrogénase (LDH).

Les résultats sont exprimés en taux de survie relatif, donné par le rapport des activités LDH, selon l'équation suivante :

$$\%\ \text{taux de survie} = \frac{LDH_{\text{cellules vieillies traitées}} - LDH_{\text{cellules témoins non traitées}}}{LDH_{\text{cellules témoins non traitées}}} * 100$$

Les valeurs figurant dans les tableaux ci-après sont des valeurs moyennes obtenues à partir de 6 mesures, après une privation de sérum de 9 jours

RESULTATS :

| | Glu-Tryp (0,86 mM) | Glu-Tryp (0,43 mM) | Glu-Tryp (0,1 mM) |
|---|---|---|---|
| amélioration du temps de survie (%) | +33 | +19 | +19 |

| | Met-Tryp (0,85 mM) | Met-Tryp (0,48) mM | Met-Tryp (0,1 mM) |
|---|---|---|---|
| amélioration du temps de survie (%) | +28 | +15 | +12 |

| | Trp-Tryp (0,85 mM) | Trp-Tryp (0,45 mM) | Trp-Tryp (0,1 mM) |
|---|---|---|---|
| amélioration du temps de survie (%) | +30 | +20 | +17 |

Test 3 : effet anti-inflammatoire de l'alpha-L-glutamyltryptamine, du L-methionyltryptamine et du L-tryptophantryptamine vis à vis de PC 12 soumises à un stress pro-inflammatoire. Comparaison avec deux témoins (PC 12), à savoir l'un non stressé, et l'autre stressé mais non traité.

Un stress UV-B pro-inflammatoire est appliqué sur les PC 12 (285 nm ± 5; 150 mJ/cm$^2$), en absence puis en présence d'actif, successivement les alpha-L-glutamyltryptamine (Glu-Tryp), L-methionyltryptamine (Met-Tryp) et L-tryptophantryptamine (Trp-Tryp).

La réponse inflammatoire neurogène est évaluée par la mesure du taux d'interleukine-6 pro-inflammatoire (IL-6) produite par les PC 12.

RESULTATS :

| | témoin non irradié | Glu-Tryp (0,86 mM) | Glu-Tryp (0,43 mM) | Glu-Try (0,1 mM) | témoin irradié non traité |
|---|---|---|---|---|---|
| taux d'IL-6 produite (pg/ml) | 0 | 70 | 180 | 240 | 400 |

| | témoin non irradié | Met-Tryp (0,85 mM) | Met-Tryp (0,48 mM) | Met-Tryp (0,1 mM) | témoin irradié non traité |
|---|---|---|---|---|---|
| taux d'IL-6 produite (pg/ml) | 0 | 85 | 210 | 290 | 400 |

| | témoin non irradié | Trp-Tryp (0,85 mM) | Trp-Tryp (0,45 mM) | Trp-Tryp (0,1 mM) | témoin irradié non traité |
|---|---|---|---|---|---|
| taux d'IL-6 produite (pg/ml) | 0 | 100 | 210 | 305 | 400 |

Test 4 : Stimulation du système neuro immuno-cutané par l'alpha-L-glutamyltryptamine, le L-methionyltryptamine ou le L-tryptophantryptamine. Comparaison avec deux témoins.

Les PC 12, différentiées selon un processus particulier pour éviter des artéfacts, c'est à dire après une brève privation en facteurs de croissance et de différentiation, sont mises en présence de concentrations variables de pseudodipeptides, successivement les alpha-L-glutamyltryptamine (Glu-Tryp), L-methionyltryptamine (Met-Tryp) et L-tryptophantryptamine (Trp-Tryp).

Après 5 jours d'incubation, les surnageants cellulaires, contenant les neuromédiateurs et autres sécrétions, sont prélevés puis introduits dans une culture de cellules monocytes immunes, la lignée THP-1.

L'effet sur le système neuro immuno-cutané est observé par la mesure du taux d'interleukine IL-1 produite par les monocytes, en réponse à l'addition des surnageants issus de la culture de PC 12.

Les résultats sont comparés à deux témoins, en l'occurence l'un comprenant les cellules immunes sans surnageant, et l'autre comprenant les cellules immunes avec surnageant mais non traitées.

1 RESULTATS:

| | THP-1 sans surnag. | THP-1 + surnag. + Glu-Tryp (0,43 mM) | THP-1 + surnag. + Glu-Tryp (0,1 mM) | THP-1 + surnag. + Glu-Tryp (0,05 mM) | THP-1 + surnage non traité |
|---|---|---|---|---|---|
| taux d'IL-1 produite (pg/ml) | 0 | 90 | 63 | 45 | 40 |

| | THP-1 sans surnag. | THP-1 + surnag. + Met-Tryp (0,48 mM) | THP-1 + surnag. + Met-Tryp (0,1 mM) | THP-1 + surnag. + Met-Tryp (0,05 mM) | THP-1 + surnag. non traité |
|---|---|---|---|---|---|
| taux d'IL-1 produite | 0 (pg/ml) | 85 | 55 | 42 | 40 |

| | THP-1 sans surnag. | Trip-1 + surnag. + Trp-Tryp P (0,45 mM) | THP-1 + surnag. + Trp-Tryp (0,1 mM) | Trip-1 + surnag. + Trp-Tryp (0,05 mM) | THP-1 + surnag. non traité |
|---|---|---|---|---|---|
| taux d'IL-1 produite (pg/ml) | 0 | 92 | 60 | 44 | 40 |

**Revendications**

1. Pseudodipeptide **caractérisé en ce qu'**il est représenté par la formule générale (I) suivante :

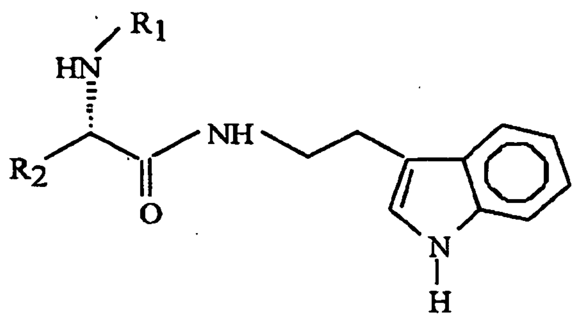

(I)

dans laquelle :

$R_1$ représente un atome d'hydrogène, un radical acyle ou alkyloxycarbonyl,
$R_2$ représente la chaîne latérale d'un alpha-aminoacide choisi parmi l'acide L-glutamique, la L-arginine, la L-méthionine, la L-histidine, le L-tryptophane, la L-tyrosine.

2. Pseudodipeptide selon la revendication 1 **caractérisé en ce qu'**il s'agit de l'alpha-L-glutamyltryptamine, du L-methionyltryptamine et du L-tryptophantryptamine.

3. Pseudodipeptide selon la revendication 1 ou 2 **caractérisé en ce qu'**il s'agit de l'alpha-L-glutamyltryptamine.

4. Pseudodipeptide selon la revendication 1 **caractérisé en ce que** $R_1$ représente un radical acétyle ou ter-butyloxy-carbonyle, $R_2$ représente la chaîne latérale d'un alpha-aminoacide choisi parmi l'acide L-glutamique, la L-méthionine, le L-tryptophane.

**5.** Procédé chimique de préparation du pseudodipeptide selon l'une quelconque des revendications 1 à 4, ledit procédé comprenant les étapes suivantes:

a) protection de la fonction alpha-amino du L-aminoacide par un radical acyle ou alkyloxycarbonyl,
b) couplage du L-aminoacide N-protégé avec la tryptamine
c) élimination ou non du groupement N-protecteur selon le pseudodipeptide recherché.

**6.** Procédé selon la revendication 5 dans lequel le L-aminoacide est acide glutamique et dans lequel l'étape a) est suivie, préalablement à l'étape b), d'une étape d'estérification de la fonction gamma-carboxylique par un radical alkyle.

**7.** Procédé selon la revendication 5 dans lequel les groupements N-protecteurs sont choisis parmi les radicaux acétyle ou ter-butyloxycarbonyle.

**8.** Composition neuro-cosmétique **caractérisée en ce qu'**elle comprend un pseudodipeptide de formule générale (I) tel que défini dans l'une des revendications 1 à 4, en association avec un ou plusieurs excipients cosmétiquement appropriés.

**9.** Utilisation d'un pseudodipeptide selon l'une quelconque des revendications 1 à 4 pour la fabrication d'un médicament présentant un effet cytoprotecteur vis-à-vis de cellules nerveuses cutanées soumises à un rayonnement ultra-violet.

**10.** Utilisation d'un pseudodipeptide selon l'une quelconque des revendications 1 à 4 pour la fabrication d'un médicament destiné à ralentir le processus de neurodégénérescence cutané par traitement local.

**11.** Utilisation d'un pseudodipeptide selon l'une quelconque des revendications 1 à 4 pour la fabrication d'un médicament destiné à lutter contre l'inflammation neurogène cutané par traitement local.

**12.** Utilisation d'un pseudodipeptide selon l'une quelconque des revendications 1 à 4 pour la fabrication d'un médicament capable de stimuler les cellules immunes cutanées.

**13.** Utilisation d'un analogue du pseudodipeptide alpha-L-glutamyltryptamine résultant de la cyclisation intramoléculaire du radical glutamique en radical pyroglutamique pour la manufacture d'un médicament destiné à ralentir le processus de neurodégénérescence cutané par traitement local.

## Claims

**1.** Pseudodipeptide **characterized in that** it has the following general formula (I) :

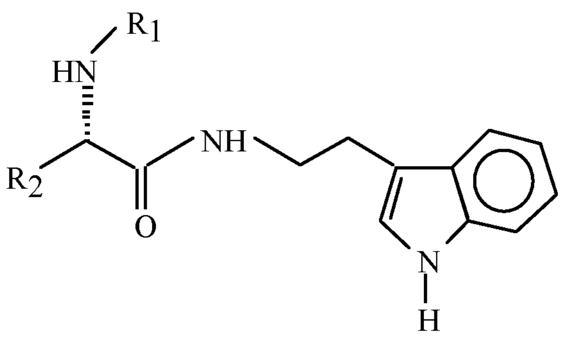

(I)

wherein :

- $R_1$ represents a hydrogen atom , an acyl or alkyloxycarbonyl radical,
- $R_2$ represents the side chain of an alpha-amino acid chosen among L-glutamic acid, L-arginine, L-methionine, L-histidine, L-tryptophan, L-tyrosine.

2. Pseudodipeptide according to claim 1, **characterized in that** it is the alpha-L-glutamyltryptamine, L-methionyltryptamine and L-tryptophantryptamine.

3. Pseudodipeptide according to claims 1 or 2, **characterized in that** it is the alpha-L-glutamyltryptamine.

4. Pseudodipeptide according to claim 1, **characterized in that** $R_1$ represents an acetyl or ter-butyloxycarbonyl radical, $R_2$ represents the side chain of an alpha-amino acid chosen among L-glutamic acid, L-methionine, L-tryptophan.

5. Chemical process for the preparation of the pseudodipeptide according to one of claims 1 to 4, the said process comprising the following steps :

   a) protection of the alpha-amino function of the L-amino acid with an acyl or alkyloxycarbonyl radical,
   b) coupling of the N-protected L-aminoacid with tryptamine,
   c) removal or not of the N-protecting group according to the targeted pseudodipeptide.

6. Process according to claim 5, wherein the L-amino acid is the glutamic acid and wherein the step a) is followed with an esterification step of the gamma-carboxylic function with an alkyl radical, prior to step b).

7. Process according to claim 5, wherein the N-protecting groups are chosen among acetyl or ter-butyloxycarbonyl radicals.

8. Neurocosmetic composition **characterized in that** it contains a pseudodipeptide of general formula (I) such as defined according to one of claims 1 to 4, in combination with one or more appropriated cosmetically excipients.

9. Use of a pseudodipeptide according to one of claims 1 to 4 for the manufacture of a medicine displaying a cytoprotecting effect towards cutaneous nervous cells submitted to ultra-violet radiation.

10. Use of a pseudodipeptide according to one of claims 1 to 4 for the manufacture of a medicine intended for slowing down the cutaneous neurodegenerescence by local treatment.

11. Use of a pseudodipeptide according to one of the claims 1 to 4 for the manufacture of a medicine intended for fighting against the cutaneous neurogenic inflammation by local treatment.

12. Use of a pseudodipeptide according to one of claims 1 to 4 for the manufacture of a medicine able to stimulate the cutaneous immune cells.

13. Use of an analog of the alpha-L-glutamyltryptamine pseudodipeptide resulting from the intramolecular cyclisation of glutamic radical in pyroglutamic radical for the manufacture of a medicine intended for slowing down the cutaneous neurodegenerescence by local treatment.

**Patentansprüche**

1. Ein Pseudodipeptid, **dadurch gekennzeichnet, dass** es durch die folgende allgemeine Formel (I) dargestellt ist:

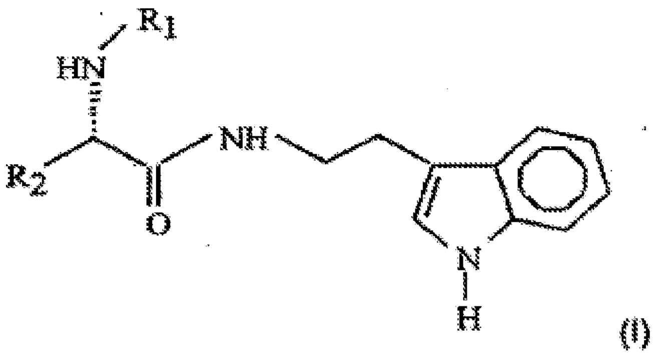

(I)

wobei:

$R_1$ ein Wasserstoffatom, ein Acylradikal oder ein Alkyloxycarbonylradikal darstellt,
$R_2$ die Seitenkette einer alpha-Aminosäure, ausgewählt aus L-Glutaminsäure, L-Argininsäure, L-Methioninsäure, L-Histidinsäure, L-Tryptophansäure, L-Tyrosinsäure, darstellt.

2. Pseudodipeptid gemäß Anspruch 1, **dadurch gekennzeichnet, dass** es sich um alpha-L-Glutamyltryptamin, L-Methionyltryptamin und L-Tryptophantryptamin handelt.

3. Pseudodipeptid gemäß Anspruch 1 oder 2, **dadurch gekennzeichnet, dass** es sich um alpha-L-Glutamyltryptamin handelt.

4. Pseudodipeptid gemäß Anspruch 1, **dadurch gekennzeichnet, dass** $R_1$ ein Acetylradikal oder ter-Butyloxycarbonylradikal darstellt, $R_2$ die Seitenkette einer alpha-Aminosäure, ausgewählt aus L-Glutaminsäure, L-Methioninsäure, L-Tryptophansäure, darstellt.

5. Ein chemisches Verfahren zur Herstellung des Pseudodipeptids gemäß einem der Ansprüche 1 bis 4, wobei das Verfahren die folgenden Schritte umfasst:

a) Schützen der alpha-Aminofunktion der L-Aminosäure mittels eines Acylradikals oder Alkyloxycarbonylradikals,
b) Koppeln der N-geschützten L-Aminosäure mit Tryptamin,
c) Beseitigen oder nicht der N-Schutzgruppe gemäß dem gesuchten Pseudodipeptid.

6. Verfahren gemäß Anspruch 5, wobei die L-Aminosäure die Glutaminsäure ist, und wobei auf Schritt a) vor Schritt b) ein Schritt der Veresterung der gamma-Carboxylfunktion durch ein Alkylradikal folgt.

7. Verfahren gemäß Anspruch 5, wobei die N-Schutzgruppen aus Acetylradikalen oder ter-Butyloxycarbonylradikalen ausgewählt sind.

8. Eine neurokosmetische Zusammensetzung, **dadurch gekennzeichnet, dass** sie ein Pseudodipeptid mit der allgemeinen Formel (I), wie in einem der Ansprüche 1 bis 4 definiert, in Verbindung mit einem oder mehreren angemessenen kosmetischen Exzipienten, umfasst.

9. Eine Verwendung eines Pseudodipeptids gemäß einem der Ansprüche 1 bis 4 zur Herstellung eines Medikaments, das eine zytoprotektive Wirkung gegenüber kutanen Nervenzellen aufweist, welche mit UV-Licht bestrahlt wurden.

10. Verwendung eines Pseudodipeptids gemäß einem der Ansprüche 1 bis 4 zur Herstellung eines Medikaments, das den Vorgang von kutaner Neurodegeneration durch lokale Behandlung verlangsamen soll.

11. Verwendung eines Pseudodipeptids gemäß einem der Ansprüche 1 bis 4 zur Herstellung eines Medikaments, das die kutane neurogene Entzündung durch lokale Behandlung bekämpfen soll.

**12.** Verwendung eines Pseudodipeptids gemäß einem der Ansprüche 1 bis 4 zur Herstellung eines Medikaments, das die kutanen Immunzellen stimulieren kann.

**13.** Eine Verwendung eines Analogs zum alpha-L-Glutamyltryptamin-Pseudodipeptid, das sich aus der intramolekularen Zyklisierung des Glutaminradikals zu einem Pyroglutaminradikal ergibt, zur Herstellung eines Medikaments, das den Vorgang der kutanen Neurodegeneration durch lokale Behandlung verlangsamen soll.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 1020179 A
- WO 9419325 A
- SU 296409


### Littérature non-brevet citée dans la description


- **Lotti T. et al.** *J. Am. Acad. Dermatol.,* 1995, vol. 33, 482-496
- **L. Misery.** *International Journal of Cosmetic Science,* 2002, vol. 24, 111-116
- **Ansel J.C. et al.** *Journal of Investigative Dermatology Symposium Proceedings,* 1997, vol. 2, 23-26
- **Takahashi K. et al.** *J. Invest. Dermatol.,* 1993, vol. 101, 646-651
- **Ahmad M. M. et al.** *JAOCS,* 1993, vol. 80, 837-840
- **Gopala Krishna A.G. et al.** *JAOCS,* 1994, vol. 71, 645-647
- **Popov I. et al.** *Luminescence,* 1999, vol. 14, 169-174
- **Burstein Y. et al.** *Proc. Natl. Acad Sci. USA,* 1976, vol. 73, 2604-2608
- **Vigy M.** *Conc. Méd.,* 1969, vol. 14, 2865-2868
- **Hindle A.T.** *Br. J. Anaesth.,* 1994, vol. 73, 395-407
- **Suvorov N.N. et al.** *Bioorg. Khim.,* 1976, vol. 2, 729-736
- **Popova G. V. et al.** *Tr. Mosk. Khim. Tekhnol. Inst. im D I Mendeleeva,* 1977, vol. 94, 84-98
- **Popova G.V. et al.** *Zh. Obshch. Khim.,* 1979, vol. 49, 1418-1424
- **Mashkovskii M.D. et al.** *Psikhiatr.,* 1963, 72
- **Vigdorchik M.M. et al.** *Pharm. Chem. J.,* 1977, vol. 11, 305-309
- **Bulatova N.N. et al.** *Khim. Farm. Zh.,* 1968, vol. 2, 6-9
- **Mc Caman M.W. et al.** *J. Neurochem.,* 1985, vol. 45, 1828-1835
- **Greene L.A. et al.** *Proc. Natl. Acad. Sci. USA,* 1976, vol. 73, 2424-2428
- **Keilbaugh S.A.** *Biochem. Pharm.,* 1997, vol. 53, 1485-1492
- **Greene L.A. et al.** Culturing Nerve Cells. MIT Press, 1991, 207-225